# EUROPEAN PATENT SPECIFICATION

(11) **EP 4 143 346 B1**
(45) Date of publication and mention of the grant of the patent: **07.01.2026**
(21) Application number: 21796694.4
(22) Date of filing: 29.04.2021
(51) Int. Cl.: C12Q 1/6886

(54) **METHOD FOR EARLY DETECTION OF COLORECTAL CANCER**
VERFAHREN ZUR FRÜHERKENNUNG VON KOLOREKTALKARZINOM
MÉTHODE DE DÉTECTION PRÉCOCE DU CANCER COLORECTAL

(30) Priority: 29.04.2020 US 202063017309 P
(43) Date of publication of application: 08.03.2023
(73) Proprietor: EG BIOMED CO., LTD., 115603, Taiwan (TW)
(72) Inventor: LIN, Ruo-Kai, New Taipei City, 221 (TW); HUNG, Chin-Sheng, Taipei City, 106 (TW); WANG, Sheng-Chao, New Taipei City, 239 (TW); LIN, Shih-Yun, New Taipei City, 235 (TW)
(74) Representative: Lavoix
(86) International application number: PCT/US2021/029965
(87) International publication number: WO 2021/222621

(56) References cited:
- WO-A1-2015/153283
- WO-A1-2017/024311
- CN-A- 110 512 000
- CN-A- 111 041 095
- US-A1- 2010 298 158
- US-A1- 2013 065 228
- VLADIMIR A NAUMOV ET AL: "Genome-scale analysis of DNA methylation in colorectal cancer using Infinium HumanMethylation450 BeadChips", EPIGENETICS, vol. 8, no. 9, 1 September 2013 (2013-09-01), US, pages 921 - 934, XP055429289, ISSN: 1559-2294, DOI: 10.4161/epi.25577
- IONESCU CALIN, BRAICU CORNELIA, CHIOREAN ROXANA, COJOCNEANU PETRIC ROXANA, NEAGOE EMILIAN, POP LAURA, CHIRA SERGIU, BERINDAN-NEAGO: "TIMP-1 expression in human colorectal cancer is associated with SMAD3 gene expression levels: a pilot study", JOURNAL OF GASTROINTESTINAL AND LIVER DISEASES, vol. 23, no. 4, 1 December 2014 (2014-12-01), pages 413 - 418, XP055862643, ISSN: 1841-8724, DOI: 10.15403/jgld.2014.1121.234.smad
- VLADIMIR A NAUMOV; EDWARD V GENEROZOV; NATALYA B ZAHARJEVSKAYA; DARYA S MATUSHKINA; ANDREY K LARIN; STANISLAV V CHERNYSHOV; MIKHAI: "Genome-scale analysis of DNA methylation in colorectal cancer using Infinium HumanMethylation450 BeadChips", EPIGENETICS, vol. 8, no. 9, 1 September 2013 (2013-09-01), pages 921 - 934, XP055429289, ISSN: 1559-2294, DOI: 10.4161/epi.25577
- SHIH-CHING CHANG; PHUI-LY LIEW; MUHAMAD ANSAR; SHIH-YUN LIN; SHENG-CHAO WANG; CHIN-SHENG HUNG; JIAN-YU CHEN; SHIKHA JAIN; RUO-KAI : "Hypermethylation and decreased expression of are potential early-onset biomarkers for colorectal cancer detection, poor prognosis, and early recurrence prediction", CLINICAL EPIGENETICS, vol. 12, no. 1, 12 May 2020 (2020-05-12), pages 1 - 17, XP021276418, ISSN: 1868-7075, DOI: 10.1186/s13148-020-00855-z

## Description

This application claims priority to and benefit of U.S. Provisional Patent Application No. 63/017,309, filed April 29, 2020.

### Field of the Invention

The disclosure relates to epigenetic biomarkers for prediction of risk or susceptibility of colorectal cancer. Particularly, the disclosure provides a method for early detection, prediction of treatment response and prognosis of colorectal cancer based on methylation statuses of gene biomarkers.

### Background of the Invention

Cancer is a group of diseases involving abnormal cell growth with the potential to invade or spread to other parts of the body and is a leading cause of deaths worldwide.

Methylated DNA has been studied as a potential class of biomarkers in the tissues of most tumor types. In many instances, DNA methyltransferases add a methyl group to DNA at cytosine-phosphate-guanine (CpG) island sites as an epigenetic control of gene expression.

US 20210003575 relates to the use of BMW Rep-protein as a biomarker for colon cancer. US 20200291479 provides methods for assessing the likelihood of effectiveness of chemotherapy such as oxaliplatin treatment in colorectal cancer patients as well as their survival prospect by determining the level of miR-133a in the cancer tissue. US 20200377959 discloses a method of detecting (e.g., screening for) colorectal cancer, the method comprising: determining a methylation status for each of the following, in deoxyribonucleic acid (DNA) of a human subject: (a) a methylation locus within gene ZNF132; (b) a first methylation locus within gene ADAMTS2; and (c) a second methylation locus within gene ADAMTS2; and diagnosing colorectal cancer in the human subject based on said determined methylation statuses. US 2013/065228 and also the publication of Vladimir A. Naumov et al., Epigenetics: vol. 8, no 9, pages 921-934 (01.09.2013) disclose the use of methylation markers for the detection of colorectal cancer.

However, the current techniques in detection of colorectal cancer are not satisfied.

### Summary of the Invention

The present disclosure discloses one or more novel epigenetic biomarkers for early detection, prediction of treatment response and prognosis of colorectal cancer. Aberrant methylation of the epigenetic biomarkers is detected in tumor tissues and plasma samples from cancer patients but not in normal individuals. The present disclosure also discloses primers and probes used herein.

In one embodiment, the present disclosure provides a method for early diagnosing colorectal cancer in a subject, comprising (a) providing a biological sample from the subject, and (b) determining the methylation status of a target DNA sequence comprising *MROH6* in the biological sample, wherein the presence of hypermethylation in *MROH6* of the subject is indicative of colorectal cancer.

In some embodiments, a target DNA sequence methylation specific probe or a target DNA sequence methylation specific primer is used to assay the methylation status of the target DNA sequence and the control DNA sequence in the biological sample.

In one embodiment, the presence of hypermethylation or hypomethylation in the target DNA sequence of the subject is determined by comparing the methylation status of the target DNA sequence to a methylation status of a control DNA sequence. In some embodiments, the present disclosure provides a method for early diagnosis of colorectal cancer in a subject, comprising (a) providing a biological sample from a subject comprising a target DNA sequence comprising *MROH6,* and (b) determining methylation statuses of the target DNA sequence and of a control DNA sequence in the biological sample using a target DNA sequence methylation specific probe or a target DNA sequence methylation specific primer, (c) measuring a relative methylation status of the target DNA sequence compared to a control DNA sequence, (d) identifying the subject as having colorectal cancer when hypermethylation is present in the relative methylation status. In some embodiments, the hypermethylation described herein is indicated when the methylation status in *TMEM240* is about 30-fold, about 32-fold, about 34-fold, about 35-fold, about 36-fold, about 37-fold, about 38-fold, about 39-fold or about 40-fold higher than that of the control DNA sequence. In a further embodiment, the methylation status in the *TMEM240* that is about 37.5-fold higher or lower than that of the control DNA sequence indicates colorectal cancer. In some embodiments, the hypermethylation described herein is indicated when the methylation status in *MROH6* is about 35-fold, about 37-fold, about 39-fold, about 40-fold, about 41-fold, about 42-fold, about 43-fold, about 44-fold, about 45-fold, about 46-fold, about 47-fold, or about 48-fold higher than that of the control DNA sequence. In a further embodiment, the methylation status in the *MROH6* that is about 44-fold higher or lower than that of the control DNA sequence indicates colorectal cancer. In a further embodiment, the control DNA sequence is in a normal tissue.

In some embodiments, the biological sample described herein is a tissue, cell, blood, urine, serum, plasma, stool, ascites, sputum, saliva, gastric juice, bile, or oral mucosa.

In some embodiments, the methylation status is detected by a polymerase chain reaction, nucleic acid sequencing (such as bisulfite sequencing or pyrosequencing), bisulfite conversion, mass spectrometry, methylation specific nuclease, mass-based separation, target capture or microarray. In a particular embodiment, the methylation status is detected by a polymerase chain reaction.

In some embodiments, the hypermethylation is indicated when a Cₜ value of the polymerase chain reaction for determining the methylation status of *TMEM240* in the human subject is less than 40, 41, 42, 43, 44, 45, 46, 47, 48, 49, or 50. In some embodiments, the hypermethylation is indicated when a Cₜ value of the polymerase chain reaction for determining the methylation status of *MROH6* in the human subject is less than 35, 36, 37, 38, 39, 40, 41, 42, 43, 44, or 45. In a preferred embodiment of the disclosure, the hypermethylation is indicated when a Cₜ value of the polymerase chain reaction for determining the methylation status of *TMEM240* in the human subject is less than 45; or the hypermethylation is indicated when a Cₜ value of the polymerase chain reaction for determining the methylation status of *MROH6* in the human subject is less than 40.

In some embodiments, the method described herein is for detecting a methylation status in a human subject who has a need of detection of colorectal cancer.

Certain embodiments of the target DNA sequence methylation specific primer used in the methylation determination for *TMEM240* has an identity of about 85%, about 86%, about 87%, about 88%, about 89%, about 90%, about 91%, about 92%, about 93%, about 94%, about 95%, about 96%, about 97%, about 98%, about 99%, or higher percent to a sequence(s) selected from the group consisting of SEQ ID NOs: 1, 2, or 3. Certain embodiments of the target DNA sequence methylation specific probe used in the methylation determination for *TMEM240* has an identity of about 85%, about 86%, about 87%, about 88%, about 89%, about 90%, about 91%, about 92%, about 93%, about 94%, about 95%, about 96%, about 97%, about 98%, about 99%, or higher percent to a sequence(s) selected from the group consisting of SEQ ID NO: 4. In some embodiments, the target DNA sequence methylation specific primer for used in the methylation determination for *MROH6* has a sequence with identity of at least 85%, about 86%, about 87%, about 88%, about 89%, about 90%, about 91%, about 92%, about 93%, about 94%, about 95%, about 96%, about 97%, about 98%, about 99% to SEQ ID NOs: 5, or 6. In some embodiments, the target DNA sequence methylation specific probe for used in the methylation determination for *MROH6* has a sequence with identity of at least 85%, about 86%, about 87%, about 88%, about 89%, about 90%, about 91%, about 92%, about 93%, about 94%, about 95%, about 96%, about 97%, about 98%, about 99% to SEQ ID NO: 7. In some embodiments, the target DNA sequence methylation specific probe for used in the methylation determination for *TMEM240* has a sequence with identity of at least 85% to a sequence selected from the group consisting of SEQ ID NOs: 1, 2, and 3. In some embodiments, the target DNA sequence methylation specific probe for *TMEM240* has an identity of about 85% to a sequence of SEQ ID NO: 4. In some embodiments, the target DNA sequence methylation specific primer for *MROH6* has an identity of about 85% to a sequence selected from the group consisting of SEQ ID NOs: 5, and 6. In some embodiments, the target DNA sequence methylation specific probe for *MROH6* has an identity of about 85% to a sequence of SEQ ID NO: 7.

In a further embodiment, the target DNA sequence further comprises one or more DNA sequences selected from the group consisting of *TMEM240, BEND5,* and *SMAD3,* or any of combinations thereof.

Certain embodiments of the target DNA sequence described herein includes any of the following combinations of the DNA sequences: *TMEM240* and *MROH6; TMEM240, MROH6* and *BEND5; TMEM240, MROH6, BEND5* and *SMAD3; TMEM240, BEND5* and *SMAD3; TMEM240, MROH6,* and *SMAD3; MROH6* and *BEND5;* and *MROH6, BEND5* and *SMAD3.* In a further embodiment, the target DNA sequences comprise *TMEM240* and *MROH6.*

In some embodiments, the methylation status is determined by a polymerase chain reaction, and the hypermethylation is indicated when a Cₜ value of the polymerase chain reaction for determining the methylation status of *BEND5* in the human subject is less than 41, 42, 43, 44, 45, 46, 47, 48, 49, or 50. In some embodiments, the methylation status is determined by a polymerase chain reaction, and the hypomethylation is indicated when a Cₜ value of the polymerase chain reaction for determining the methylation status of *SMAD3* in the human subject is higher than 30, 31, 32, 33, 34, 35, 36, 37, 38, 39, or 40. In some embodiments of the disclosure, the hypermethylation is indicated when a Cₜ value of the polymerase chain reaction for determining the methylation status of *BEND5* in the human subject is less than 45. In some embodiments, the hypomethylation is indicated when a Cₜ value of the polymerase chain reaction for determining the methylation status of *SMAD3* in the human subject is higher than 45.

In some embodiments of the disclosure, the method further comprising a step of defining a score as 1 if the presence of hypermethylation or hypomethylation of each gene and, defining a score as 0 if the absence of hypermethylation or hypomethylation of each gene, and summing the score.

In some embodiments of the disclosure, the method further comprises:
defining a score for *TMEM240* as 1, if a Cₜ value of *TMEM240* is less than 45; defining a score for *TMEM240* as 0, if a Cₜ value of *TMEM240* is higher than or equal to 45;
defining a score for *MROH6* as 1, if a Cₜ value of *MROH6* is less than 40; defining a score for *MROH6* as 0, if a Cₜ value of *MROH6* is higher than or equal to 45;
defining a score for *BEND5* as 1, if a Cₜ value of *BEND5* is less than 45, and summing the score;
defining a score for *BEND5* as 0, if a Cₜ value of *BEND5* is higher than or equal to 45; or
defining a score for *SMAD3* as 1, if a Cₜ value of *SMAD3* is higher than 45; defining a score for *SMAD3* as 0, if a Cₜ value of *SMAD3* is less than or equal to 45; and
summing the scores for *TMEM240, MROH6, BEND5* and *SMAD3.*

In some embodiments of the disclosure, if the sum of the sores for *TMEM240, MROH6,* and *BEND5* is higher than 0.15, 0.16, 0.17, 0.18, 0.19, 0.20, 0.21, 0.22, 0.23, 0.24, or 0.25, the subject is indicative of colorectal cancer. In some embodiments of the disclosure, if the sum of the sores for *TMEM240, MROH6,* and *BEND5* is higher than 0.20, the subject is indicative of colorectal cancer.

In some embodiments, a target DNA sequence methylation specific probe or a target DNA sequence methylation specific primer or any of combinations thereof, as described herein, are further used for determining a methylation status of the following one or more DNA sequences or any of combinations thereof: *BEND5* and *SMAD3.*

In some embodiments, the target DNA sequence methylation specific primer for *BEND5* described herein has a sequence with identity of at least 85%, 88%, 90%, 91%, 92%, 93%, 94%, 95%, 96%, 97%, 98% or 99% to SEQ ID NO: 8, 9, 10 or 11. In some further embodiments, the target DNA sequence methylation specific primer for *BEND5* has a sequence of SEQ ID NO: 8, 9, 10 or 11. The target DNA sequence methylation specific probe for *BEND5* has a sequence with identity of at least 85%, 88%, 90%, 91%, 92%, 93%, 94%, 95%, 96%, 97%, 98% or 99% to SEQ ID NO: 12 or 13. In a further embodiment, the target DNA sequence methylation specific probe for *BEND5* has a sequence of SEQ ID NO: 12 or 13.

In some embodiments, the target DNA sequence methylation specific primer for *SMAD3* described herein has a sequence with identity of at least 85%, 88%, 90%, 91%, 92%, 93%, 94%, 95%, 96%, 97%, 98% or 99% to SEQ ID NO: 14 or 15. In some further embodiments, the target DNA sequence methylation specific primer for *SMAD3* has a sequence of SEQ ID NO: 14 or 15. The target DNA sequence methylation specific probe for *SMAD3* has a sequence with identity of at least 85%, 88%, 90%, 91%, 92%, 93%, 94%, 95%, 96%, 97%, 98% or 99% to SEQ ID NO: 16. In a further embodiment, the target DNA sequence methylation specific probe for *SMAD3* has a sequence of SEQ ID NO: 16.

In a further embodiment, determining the methylation status further comprises a step of measuring the specificity and sensitivity by a weighted sum score analysis. In some further embodiments, determining the methylation status of the combination of target *TMEM240, MROH6, BEND5* and *SMAD3* or target *TMEM240, MROH6, BEND5* and *SMAD3* has about 100% of sensitivity and about 100% specificity and about 100% of accuracy.

In a further embodiment, the method described herein further comprises a step of administering an anti-colorectal cancer agent to the subject.

The present disclosure provides a method for detecting a predisposition to colorectal cancer or predicting likelihood, treatment response, prognosis or recurrence of colorectal cancer in a subject, comprising (a) providing a biological sample from the subject, and (b) determining a methylation status of a target DNA sequence comprising *TMEM240* and *MROH6* in the biological sample; wherein the presence of hypermethylation or hypomethylation in the target DNA sequence of the subject is indicative of colorectal cancer.

In some embodiments of the disclosure, the method comprises determining the methylation status of *TMEM240* using a target DNA sequence methylation specific primer having a sequence with identity of at least 85% to SEQ ID NO: 1, 2, or 3 or a target DNA sequence methylation specific probe having a sequence with identity of at least 85% to SEQ ID NO: 4, and determining a methylation status of *MROH6* using a *MROH6* methylation specific primer having a sequence with homology of at least 85% to SEQ ID NO: 5 or 6 or a *MROH6* sequence methylation specific probe having a sequence with homology of at least 85% to SEQ ID NO: 7.

In some embodiments of the disclosure, the target DNA sequence further comprises one or more DNA sequences selected from the group consisting of *BEND5* and *SMAD3,* or any of combinations thereof.

The present disclosure provides an isolated nucleic acid molecule having a sequence selected from the group consisting of: SEQ ID Nos: 1 to 16.

The present disclosure also provides a kit for detecting a predisposition to colorectal cancer or predicting likelihood, treatment response, prognosis or recurrence of colorectal cancer in a subject, which comprises an isolated nucleic acid molecule for assaying a methylation status of the target DNA sequence as described herein. The kit may further comprise sodium bisulfite and adapters for whole target genes amplification, and polynucleotides (e.g., detectably-labeled polynucleotides) to quantify the presence of a methylated and/or an unmethylated cytosine residue in the target DNA sequence in target DNA sequence as described herein. Furthermore, the kit may further comprise a methylation sensing restriction enzyme for whole target sequence or genes amplification.

The present disclosure provides a target DNA sequence methylation specific primer pair for detection of a methylation status of *TMEM240,* comprising SEQ ID NOs: 1 and 2 or a sequence having at least 85% identity thereof; or SEQ ID NOs: 1 and 3 or a sequence having at least 85% identity thereof. The present disclosure provides a target DNA sequence methylation specific probe for detection of a methylation status of *TMEM240,* comprising SEQ ID NO: 4 or a sequence having at least 85% identity thereof.

The present disclosure provides a target DNA sequence methylation specific primer pair for detection of a methylation status of *MROH6,* comprising SEQ ID NOs: 5 and 6 or a sequence having at least 85% identity thereof. The present disclosure provides a target DNA sequence methylation specific probe for detection of a methylation status of *MROH6,* comprising SEQ ID NO: 7 or a sequence having at least 85% identity thereof.

The present disclosure provides a target DNA sequence methylation specific primer pair for detection of a methylation status of *BEND5,* comprising SEQ ID NOs: 8 and 9 or a sequence having at least 85% identity thereof; or SEQ ID NOs: 10 and 11 or a sequence having at least 85% identity thereof. The present disclosure provides a target DNA sequence methylation specific probe for detection of a methylation status of *BEND5,* comprising SEQ ID NO: 12 or 13 or a sequence having at least 85% identity thereof.

The present disclosure provides a target DNA sequence methylation specific primer pair for detection of a methylation status of *SMAD3,* comprising SEQ ID NOs: 14 and 15 or a sequence having at least 85% identity thereof. The present disclosure provides a target DNA sequence methylation specific probe for detection of a methylation status of *SMAD3,* comprising SEQ ID NO: 16 or a sequence having at least 85% identity thereof.

The present disclosure also discloses a kit for detecting a predisposition to colorectal cancer or predicting likelihood, treatment response, prognosis or recurrence of colorectal cancer in a subject, which comprises a target DNA sequence methylation specific primer pair for detection of a methylation status of a target DNA sequence comprising *TMEM240*and *MROH6* or fragment thereof. In one embodiments of the disclosure, the kit further comprises a target DNA sequence methylation specific probe for detection of a methylation status of a target DNA sequence comprising *TMEM240*and *MROH6* or fragment thereof.

In some embodiments, the kit further comprises one or more target DNA sequence methylation specific primer pairs for detection of a methylation status of *BEND5*or *SMAD3,* or any combination thereof. In some embodiments, the kit further comprises one or more target DNA sequence methylation specific probe for detection of a methylation status of *BEND5* or *SMAD3,* or any combination thereof.

### Brief Description of the Drawings

Figures 1A to 1H show the heatmap for difference of methylation statuses of target nucleic acid and promoter, exon and gene body region of genes between tumor tissues and adjacent normal tissues, respectively (FIG. 1A: TMEM240 of Taiwan samples; FIG. 1B: MROH6 of Taiwan samples; FI. 1C: BEND5 of Taiwan samples; FIG. 1D: SMAD3 of Taiwan samples; FIG 1E: TMEM240 of TCGA samples; FIG. 1F: MROH6 of TCGA samples; FIG. 1G: BEND5 of TCGA samples; FIG. 1H: SMAD3 of TCGA samples).
Figures 2A to 2E shows the difference in early detection of the methylation status of epigenetic biomarkers of target genes in plasma samples of healthy subjects and colorectal cancer patients (FIG. 2A: TMEM240; FIG. 2B: MROH6; FIG. 2C: BEND5; FIG. 2D: SMAD3; FIG. 2E: the DNA methylation levels forTMEM240; MROH6 and BEND5).
Figure 3 shows the sum of DNA methylation levels (scores) of epigenetic biomarkers of target genes in plasma samples of healthy subjects and colorectal cancer patients.
Figure 4 shows Receiver Operating Characteristic (ROC) Curve Analysis exhibiting the early detection of the methylation status of epigenetic biomarkers of target genes in colorectal cancer patients and healthy subjects.

### Detailed Description of the Invention

It is understood that this invention is not limited to the particular materials and methods described herein. It is also to be understood that the terminology used herein is for the purpose of describing particular embodiments and is not intended to limit the scope of the present invention, which will be limited only by the appended claims.

It must be noted that, as used in this specification and the appended claims, the singular forms "a," "an," and "the" include plural referents unless the content clearly dictates otherwise. Thus, for example, reference to "a biomarker" includes a mixture of two or more biomarkers, and the like.

The term "AUC" as used herein is an abbreviation for the area under a curve. In particular it refers to the area under a Receiver Operating Characteristic (ROC) curve. The ROC curve is a plot of the true positive rate against the false positive rate for the different possible cut points of a diagnostic test. It shows the trade-off between sensitivity and specificity depending on the selected cut point (any increase in sensitivity will be accompanied by a decrease in specificity). The area under an ROC curve (AUC) is a measure for the accuracy of a diagnostic test (the larger the area the better, optimum is 1, a random test would have a ROC curve lying on the diagonal with an area of 0.5; for reference: J. P. Egan. Signal Detection Theory and ROC Analysis, Academic Press, New York, 1975).

The term "a biological sample" refers to a sample of tissue, cells, or fluid isolated from a subject, including but not limited to, for example, blood, buffy coat, plasma, serum, blood cells (e.g., peripheral blood mononucleated cells (PBMCS), band cells, neutrophils, metamyelocytes, monocytes, or T cells), fecal matter, urine, bone marrow, bile, stool, ascites, sputum, spinal fluid, lymph fluid, samples of the skin, external secretions of the skin, respiratory, intestinal, and genitourinary tracts, tears, saliva, milk, organs, biopsies and also samples of in vitro cell culture constituents, including, but not limited to, conditioned media resulting from the growth of cells and tissues in culture medium, *e.g*., recombinant cells, and cell components.

The term "a biomarker" refers to a nucleic acid molecule which is present in a sample taken from patients having human cancer as compared to a comparable sample taken from control subjects (e.g., a person with a negative diagnosis or undetectable cancer, normal or healthy subject). The biomarker can be a nucleic acid, a fragment of a nucleic acid, a polynucleotide, or an oligonucleotide that can be detected and/or quantified. Biomarkers include polynucleotides comprising nucleotide sequences from genes.

The term "a CpG island" as used herein refers to stretches of DNA in a genome that are rich in GC relative to the rest of the genome. Typically, the GC content is 50% or greater in these regions, which extend over hundreds of base pairs and sometimes thousands. Often these regions mark the 5' ends of genes.

As used herein, the term "early detection" of cancer refers to discovering the likelihood of cancer before metastasis. Preferably, it refers to discovering the likelihood of cancer before a morphological change in a sample tissue or cell is observed.

As used herein, the terms "detect", "detecting" or "detection" may describe either the general act of discovering or discerning or the specific observation of a detectably labeled composition.

The term "gene" refers to a nucleic acid (e.g., DNA) sequence that comprises coding sequences necessary for the production of a polypeptide, precursor, or RNA (e.g., non-coding RNAs such as ribosomal RNA, transfer RNA, splicosomal RNA, microRNA). A polypeptide or non-coding RNA can be encoded by a full-length coding sequence or by any portion of the coding sequence so long as the desired activity or functional properties (e.g., enzymatic activity, ligand binding, signal transduction, immunogenicity, etc.) of the full-length or fragment polypeptide are retained. Accordingly, a gene can include or exclude promoter sequences, terminators, translational regulatory sequences such as ribosome binding sites and internal ribosome entry sites, enhancers, silencers, insulators, boundary elements, replication origins, matrix attachment sites and locus control regions. The term also encompasses the coding region of a structural gene and the sequences located adjacent to the coding region on both the 5' and 3' ends for a distance of about 1 kb or more on either end such that the gene corresponds in length to the full-length mRNA. The term "gene" further includes both cDNA and genomic forms of a gene.

As used herein, the term "promoter" refers to a region of DNA that generally is located upstream (towards the 5' region of a gene) of a gene and is needed to initiate and drive transcription of the gene. A promoter may permit proper activation or repression of a gene that it controls. A promoter may contain specific sequences that are recognized by transcription factors. These factors may bind to a promoter DNA sequence, which results in the recruitment of RNA polymerase, an enzyme that synthesizes RNA from the coding region of the gene. The promoter generally refers to all gene regulatory elements located upstream of the gene, including, upstream promoters, 5' UTR, introns, and leader sequences.

The term "exon" refers to any segment of an interrupted gene that is represented in a mature RNA product. The term "intron" refers to any segment of DNA that is transcribed but removed from within the transcript by splicing together the exons on either side of it. Operationally, exon sequences occur in the mRNA sequence of a gene. Operationally, intron sequences are the intervening sequences within the genomic DNA of a gene, bracketed by exon sequences and usually having GT and AG splice consensus sequences at their 5' and 3' boundaries.

As used herein, the term "homology" refers to a first sequence which shares a degree of sequence identity with a second sequence, but whose sequence is not identical to that of the second sequence. For example, a polynucleotide comprising the wild-type sequence of a mutant gene is homologous and non-identical to the sequence of the mutant gene. In some embodiments, the degree of homology between the two sequences is sufficient to allow homologous recombination therebetween, under appropriate stringent conditions.

Techniques for determining nucleic acid and amino acid sequence identity include determining the nucleotide sequence of the mRNA for a gene and/or determining the amino acid sequence encoded thereby, and comparing these sequences to a second nucleotide or amino acid sequence. Genomic sequences can also be determined and compared in this fashion. In general, identity refers to an exact nucleotide-to-nucleotide or amino acid-to-amino acid correspondence of two polynucleotides or polypeptide sequences, respectively. Two or more sequences (polynucleotide or amino acid) can be compared by determining their percent identity. The percent identity of two sequences, whether nucleic acid or amino acid sequences, is the number of exact matches between two aligned sequences divided by the length of the shorter sequences and multiplied by 100.

In some embodiments, the degree of sequence similarity between polynucleotides can be determined by hybridization of polynucleotides under conditions that allow formation of stable duplexes between homologous regions, followed by digestion with single-stranded-specific nuclease(s), and size determination of the digested fragments. Two nucleic acid, or two polypeptide sequences are substantially homologous to each other when the sequences exhibit at least about 70%-75%, preferably 80%-82%, more preferably 85%-90%, even more preferably 92%, still more preferably 95%, and most preferably 98% sequence identity over a defined length of the molecules, as determined using the methods above. As used herein, substantially homologous also refers to sequences showing complete identity to a specified DNA or polypeptide sequence. DNA sequences that are substantially homologous can be identified in a Southern hybridization experiment under, for example, stringent conditions, as defined for that particular system. *See,* e.g., Sambrook et al., supra; Nucleic Acid Hybridization: A Practical Approach, editors B. D. Hames and S. J. Higgins, (1985) Oxford; Washington, D.C.; IRL Press).

As used herein, the term "prediction" refers to the likelihood that a patient will respond either favorably or unfavorably to a drug or set of drugs, and also the extent of those responses. Thus, treatment predictive factors are variables related to the response of an individual patient to a specific treatment, independent of prognosis.

The term "methylation," as used herein, refers to the presence of a methyl group added by the action of a DNA methyl transferase enzyme to a cytosine base or bases in a region of nucleic acid, e.g., genomic DNA.

The term "methylation status" of a nucleic acid molecule refers to the presence or absence of one or more methylated nucleotide bases in the nucleic acid molecule. For example, a nucleic acid molecule containing a methylated cytosine is considered methylated (i.e., the methylation state of the nucleic acid molecule is methylated). A nucleic acid molecule that does not contain any methylated nucleotides is considered unmethylated.

The term "hypermethylation" refers to the average methylation state corresponding to an increased presence of methylated nucleotide bases in the nucleic acid molecule at one or a plurality of CpG dinucleotides within a DNA sequence of a test DNA sample, relative to the amount of methylated nucleotide bases in the nucleic acid molecule found at corresponding CpG dinucleotides within a normal control DNA sample.

The term "hypomethylation" refers to the average methylation state corresponding to a decreased presence of methylated nucleotide bases in the nucleic acid molecule at one or a plurality of CpG dinucleotides within a DNA sequence of a test DNA sample, relative to the amount of methylated nucleotide bases in the nucleic acid molecule found at corresponding CpG dinucleotides within a normal control DNA sample.

The term "a Cₜ value" is the abbreviation for threshold cycle and is defined as the calculated cycle number at which the PCR product crosses a threshold of detection.

The term "subject" refers to humans.

The term "susceptibility" refers to a constitution or condition of the body which makes the tissues react in special ways to certain extrinsic stimuli and thus tends to make the individual more than usually susceptible to certain diseases.

The term "target site" or "target sequence" refers to a nucleic acid sequence that defines a portion of a nucleic acid to which a binding molecule will bind, provided sufficient conditions for binding exist.

The term "risk" refers to the estimated chance of getting a disease during a certain time period, such as within the next 10 years, or during the subject's lifetime.

The term "prognosis" as used herein generally refers to a prediction of the probable course and outcome of a clinical condition or disease. A prognosis of a patient is usually made by evaluating factors or symptoms of a disease that are indicative of a favorable or unfavorable course or outcome of the disease.

The term "weight sum score" refers to every possible alternative being rated by a score including all objectives, individually weighted to stress the importance of different objectives.

As used herein, the term "nucleic acid molecule" (or "nucleic acid" or "polynucleotide") may refer to a polymeric form of nucleotides, which may include both sense and anti-sense strands of RNA, cDNA, genomic DNA, and synthetic forms and mixed polymers of the above. A nucleotide may refer to a ribonucleotide, deoxyribonucleotide, or a modified form of either type of nucleotide. A "nucleic acid molecule" as used herein is synonymous with "nucleic acid" and "polynucleotide". A nucleic acid molecule is usually at least 10 bases in length, unless otherwise specified. The term may refer to a molecule of RNA or DNA of indeterminate length. The term includes single- and double-stranded forms of DNA. A nucleic acid molecule may include either or both naturally-occurring and modified nucleotides linked together by naturally occurring and/or non-naturally occurring nucleotide linkages.

Cancer is characterized by an abnormal growth of a cell caused by one or more mutations or modifications of a gene leading to dysregulated balance of cell proliferation and cell death. In many disease processes, such as cancer, gene promoter CpG islands acquire abnormal hypermethylation, which results in transcriptional silencing that can be inherited by daughter cells following cell division. DNA methylation causing silencing in cancer typically occurs at multiple CpG sites in the CpG islands that are present in the promoters of protein coding genes. Alterations of DNA methylation have been recognized as an important component of cancer development. DNA methylation profiling provides higher clinical sensitivity and dynamic range compared to other cancer detections. Accordingly, the present disclosure provides a method and kit for early prediction, treatment response and prognosis or recurrence monitoring of colorectal cancer.

In the present disclosure, the methylation status of target DNA sequences in a biological sample are measured to detect colorectal cancer in a human subject. In further embodiments, the methylation status of a target DNA sequence comprising *MROH6* in a biological sample are measured to detect colorectal cancer in a human subject. In a further embodiment, the methylation status of *TMEM240, BEND5* and *SMAD3* is further measured.

*TMEM240* encodes a transmembrane domain-containing protein transmembrane protein 240, found in the brain and cerebellum. Mutations of *TMEM240* were found to cause spinocerebellar ataxia 21 (SCA21) with mental retardation, severe cognitive impairment, and hypokinetic and hyperkinetic movement disorders. In one preferred embodiment, the target DNA sequence comprises the promoter and exon 1 regions of *TMEM240.*

*MROH6* gene encodes maestro heat like repeat family member 6. Diseases associated with MROH6 include Non-Syndromic Intellectual Disability and Autosomal Recessive Non-Syndromic Intellectual Disability.

*BEND5* gene encodes BEN Domain Containing 5, which acts as a transcriptional repressor. In one preferred embodiment, the target DNA sequence comprises the promoter and exon 1 regions of *BEND5.*

*SMAD3* gene encodes SMAD Family Member 3, which is related to the transforming growth factor-β. In one preferred embodiment, the target DNA sequence comprises the promoter regions of *SMAD3.*

In some embodiments, the methylation comprises a cytosine methylation site. In some instances, cytosine methylation comprises 5-methylcytosine (5-mCyt) and 5-hydroxymethylcytosine. In some cases, a cytosine methylation site occurs in a CpG dinucleotide motif. In other cases, a cytosine methylation site occurs in a CHG or CHH motif, in which is adenine, cytosine or thymine. In some instances, one or more CpG dinucleotide motif or CpG site forms a CpG island, a short DNA sequence rich in CpG dinucleotide. In some instances, CpG islands are typically, but not always, between about 0.2 to about 1 kb in length. In some instances, the methylation comprises CpG island methylation.

In some embodiments, the methylation status is analyzed by a methylation specific enzymatic digest; bisulfite sequencing; an analysis selected from promoter methylation, CpG island methylation, MSP, HeavyMethyl, MethyLight, and Ms-SNuPE; and other methods relying on a detection of amplified DNA. The term "MethyLight^{™}" refers to a fluorescence-based real-time PCR technique. MethylLight is described by Eads et al., Cancer Res. 59:2302-2306, 1999.

The term "HeavyMethyl" assay, refers to an assay wherein methylation specific blocking probes (also referred to herein as blockers) covering CpG positions between, or covered by the amplification primers enable methylation-specific selective amplification of a nucleic acid sample.

The term "Ms-SNuPE" refers to Methylation-sensitive Single Nucleotide Primer Extension. MsSNuPE is described by Gonzalgo & Jones, Nucleic Acids Res. 25:2529-2531, 1997.

The term "MSP" refers to Methylation-specific PCR. MSP is described by Herman et al. Proc. Natl. Acad. Sci. USA 93:9821-9826, 1996, and by U.S. Pat. No. 5,786,146.

Bisulfite modification of DNA is a method to assess CpG methylation status. 5-methylcytosine is the most frequent covalent base modification in the DNA of eukaryotic cells. However, 5-methylcytosine positions cannot be identified directly by sequencing or hybridization methods, because 5-methylcytosine has the same base pairing behavior as cytosine. Moreover, the epigenetic information carried by 5-methylcytosine is completely lost during, *e.g.,* PCR amplification. Bisulfite sequencing is a method for analyzing DNA for the presence of 5-methylcytosine is based upon the specific reaction of bisulfite with cytosine whereby, upon subsequent alkaline hydrolysis, cytosine is converted to uracil which corresponds to thymine in its base pairing behavior. However, 5-methylcytosine remains unmodified under the aforementioned conditions. Thus, the original DNA is converted in such a manner that methylcytosine, which originally could not be distinguished from cytosine by its hybridization behavior, can be detected as the only remaining cytosine using molecular biological techniques, for example, by amplification and hybridization, or by sequencing.

In one embodiment, the methylation status is detected by polymerase chain reaction, nucleic acid sequencing (such as bisulfite sequencing or pyrosequencing), bisulfite conversion, mass spectrometry, methylation specific nuclease, mass-based separation, target capture or microarray. In one embodiment, the methylation status is detected by using primers to amplify a methylated CpG of the target genes. In a further embodiment, the detection of methylation is conducted by PCR, methylation specific PCR (MSP), real-time methylation specific PCR, quantitative methylation-specific PCR (QMSP), PCR using a methylated DNA-specific binding protein or quantitative PCR.

In one embodiment of the present disclosure, a target DNA sequence methylation specific primer that could amplify a methylated CpG of the genes described herein might be used. The target DNA sequence methylation specific primer comprises at least one or more CpG dinucleotide in a region which hybridizes to the methylated CpG of the genes. Specifically, the target DNA sequence methylation specific primer for amplifying a methylated CpG of the genes comprise sequence having a homology of about 85%, about 86%, about 87%, about 88%, about 89%, about 90%, about 91%, about 92%, about 93%, about 94%, about 95%, about 96%, about 97%, about 98%, about 99%, or higher percent to sequence(s) selected from the group consisting of the following sequences as shown in Table 1.

In one embodiment of the present disclosure, a target DNA sequence methylation specific probe capable of hybridizing with a methylated CpG of the genes described herein might be used. The target DNA sequence methylation specific probe capable of hybridizing with a methylated CpG of the genes comprise at least one or more CpG dinucleotide in a region which hybridizes to the methylated CpG of the genes. Specifically, probe(s) might comprise sequence(s) having a homology of about 85%, about 86%, about 87%, about 88%, about 89%, about 90%, about 91%, about 92%, about 93%, about 94%, about 95%, about 96%, about 97%, about 98%, about 99%, or higher percent to sequence(s) selected from the group consisting of the following sequences as shown in Table 1.

**Table 1**

| SEQ ID NO. | Primer or Probe | Sequence |
|---|---|---|
| 1 | TMEM240-qMSP-F | TTTAGAATTATGAAGATTATGGTGTTC |
| 2 | TMEM240-qMSP-R | AAAACTCAACATCGAACCGA |
| 3 | TMEM240-qMSP-R2 | CGACCCCGCCCGATATCCATAA |
| 4 | TMEM240-qMSP-probe | TTTAGAATTATGAAGATTATGGTGTTC |
| 5 | MROH6-qMSP-F2 | GGTGAGTTTTTGATTTGTAATTGTC |
| 6 | MROH6-qMSP-R | ATCTCGTACCGCTACTACTACGC |
| 7 | MROH6-qMSP-probe | GTCGGGGGTTGTTGATTTTAGTAGCGTT |
| 8 | BEND5-qMSP-F2 | GTTTGGGTTTTGGGGAGTC |
| 9 | BEND5-qMSP-R | GATCGAACAACTCAACCCG |
| 10 | BENDS-forward | GTT TTT GTG CGG TTT TTG GA |
| 11 | BENDS-reverse | AAC CGC GAA CGA AAA CTA AA |
| 12 | BEND5-qMSP-probe | CGAAAATAAAAATCCGACGA |
| 13 | BENDS-probe | TT GTT ACG CG TTG TTC GTG T |
| 14 | SMAD3-qMSP-F | GAATAAGGTCGTTAGTTATTATCGT |
| 15 | SMAD3-qMSP-R | AATCAAATCTACCCGAATCGAA |
| 16 | SMAD3-qMSP-probe | GAAAGAAAGAAAGAAAGTAAATTTTATTTTTA AGCG |

In one embodiment, the detection of the methylation status of the target DNA sequence comprises the presence of hypermethylation in the target DNA sequence relative to a normal state of the target genes.

In some embodiments, the biological sample is a tissue, cell, blood, urine, serum, plasma, stool, ascites, sputum, saliva, gastric juice, bile, or oral mucosa from a human subject suspected of having colorectal cancer or a human subject to be detected.

As used herein, the term "who is in a need of detection of cancer" refers to an individual who has received an initial diagnosis (*e.g*., a CT scan showing a mass or increased biomarker level) but for whom the stage of cancer or presence or absence of methylated genes indicative of cancer is not known. The term further includes people who once had cancer (*e.g.,* an individual in remission).

In some embodiments, a detection test to correctly predict status is measured as the sensitivity of the assay, the specificity of the assay or the area under a receiver operated characteristic (ROC) curve (AUC). The greater the area under the ROC curve, for example, the more accurate or powerful the predictive value of the test.

In one embodiment, a weighted sum score is measured to determine the methylation status in the nucleic acid sequence and genes as an indicator. The weighted sum model (WSM) is the best known and simplest multi-criteria decision analysis (MCDA)/multi-criteria decision making method for evaluating a number of alternatives in terms of a number of decision criteria. According to the present disclosure, the weighted sum score analysis shows the combination of *TMEM240, MROH6, BEND5* and *SMAD3* shows a sensitivity of about 100% and a specificity of about 96% than the control.

In some embodiments, one or more of the biomarkers disclosed herein show a statistical difference in different samples of at least p<0.05. Detection tests that use these biomarkers may show an AUC of at least 0.9.

In some embodiments, the present disclosure provides an isolated nucleic acid molecule having a sequence selected from the group consisting of: SEQ ID Nos: 5 to 7.

In some embodiments, the present disclosure provides a kit for early diagnosing colorectal cancer in a subject, which comprises a target DNA sequence methylation specific primer pair for detection of a methylation status of a target DNA sequence comprising *MROH6,* wherein the target DNA sequence methylation specific primer pair for *MROH6* comprises SEQ ID NOs: 5 and 6.

In some preferred embodiments of the disclosure, the kit further comprises a target DNA sequence methylation specific probe for detection of a methylation status of a target DNA sequence comprising *MROH6,* wherein the target DNA sequence methylation specific probe for *MROH6* comprises SEQ ID NO: 7. In some preferred embodiments of the disclosure, the kit further comprises one or more target DNA sequence methylation specific primer pairs for detection of a methylation status of *TMEM240, BEND5,* or *SMAD3,* or any combination thereof; wherein the target DNA sequence methylation specific primer pair for *TMEM240* comprises SEQ ID NOs: 1 and 2 or 1 and 3; the target DNA sequence methylation specific primer pair for *BEND5* comprises SEQ ID Nos. 8 and 9 or 10 and 11; and the target DNA sequence methylation specific primer pair for *SMAD3* comprises SEQ ID Nos. 14 and 15. In some preferred embodiments of the disclosure, the kit further comprises one or more target DNA sequence methylation specific probe for detection of a methylation status of *TMEM240, BEND5,* or *SMAD3,* or any combination thereof, and wherein the target DNA sequence methylation specific probe for *TMEM240* comprises SEQ ID NO: 4; the target DNA sequence methylation specific probe for *BEND5* comprises SEQ ID NO: 12 or 13; and the target DNA sequence methylation specific probe for *SMAD3* comprises SEQ ID NO: 16.

### EXAMPLE

### Materials and Methods

### Sample Preparation

Blood samples were collected using an ETDA-K2 tube and PAXgene Blood ccfDNA (circulating cell-free DNA) tube (Qiagen, Hilden, Germany, 768165) designed specifically for in vitro diagnostic ccfDNA testing. The samples collected using ETDA-K2 tube (BD, Plymouth, UK, 367525) were immediately centrifuged at 2000× g for 10 min at 4 °C. Within 2 h, the supernatant from each sample was transferred to a new centrifuge tube and centrifuged at 6000× g for 30 min at 4 °C and subsequently stored at -80 °C. Samples collected using the PAXgene Blood ccfDNA tube were kept at room temperature (15-25 °C) until use within 3 days, and subsequently centrifuged at 2000× g for 10 min at 4 °C followed by 6000× g for 30 min at 4 °C for plasma separation. The plasma of each sample was split into 1.6 mL aliquots and immediately frozen at -80 °C until further use.

### The Cancer Genome Atlas Portal

The data of the Western cohort are based on data generated by The Cancer Genome Atlas (TCGA) Research Network from Genomic Data Commons (GDC) data portal. The Cancer Genome Atlas (TCGA) is a collaboration between the National Cancer Institute (NCI) and the National Human Genome Research Institute (NHGRI) that has generated comprehensive, multidimensional maps of the key genomic changes in 33 types of cancer. The TCGA dataset, comprising more than two petabytes of genomic data, is now accessible to the cancer research community to improve the prevention, diagnosis and treatment of cancer.

### Genomic DNA Extraction

Genomic DNA from matched pairs of primary tumors and adjacent colorectal tissues from the same patient was extracted using the QIAamp DNA Mini Kit (Qiagen, Bonn, Germany, Cat. No. 51306) according to manufacturer's instruction. After DNA quantification, the purity was verified by measuring the A260/A280 ratio (range 1.8 to 2.0) using a NanoDrop ND-1000 spectrophotometer (NanoDrop Technologies Inc, Wilmington, DE, USA).

### Manual Circulating Cell-Free DNA Extraction

Circulating cell-free DNA (cfDNA) from plasma samples was extracted using the MagMAX Cell-Free DNA Isolation Kit (Thermo Fisher Scientific, Austin, TX, USA) or Catch-cfDNA Serum/Plasma kit (CatchGene, New Taipei City, Taiwan, R.O.C.) according to the manufacturer's recommended protocol. The ccfDNA samples had clear fragment size peaks between 140 and 200 bp. The DNA Isolation kit provided the highest yield and low molecular weight fractions. The plasma was isolated immediately from 10 mL of peripheral blood within 2 h. After DNA quantification, the purity was verified by measuring the A260/A280 ratio (range 1.8 to 2.0) using a NanoDrop ND-1000 spectrophotometer (NanoDrop Technologies, Inc., Wilmington, DE, USA).

### Automatic Circulating Cell-Free DNA Extraction and Bisulfite Conversion by KingFisher^{™} Duo Prime

An automated process for ccfDNA extraction and bisulfite conversion on the KingFisher^{™} Duo Prime purification system (ThermoFisher Scientific, Singapore) was applied according to the manufacturer's instructions. This process fully automates magnetic bead-based DNA extraction of up to six samples simultaneously. The workflow was adapted as described in the instruction manual supplied with the MagMAX^{™} cell-free DNA isolation kit (ThermoFisher Scientific, Austin, TX, USA, A29319). The ccfDNA was extracted from 1.6 mL of plasma and eluted in 60 µL of molecular biology-grade water (Corning, NY, USA, 46-000-CM). The bisulfite conversion cleanup was also performed on this machine for the semiautomatic assay. The automated protocol for the bisulfite conversion cleanup was developed with the instruction manual supplied with the EZ-96 DNA Methylation-Lightning^{™} MagPrep Kit (Zymo Research, Irvine, CA, USA, D5046). The extracted ccfDNA was incubated with sodium bisulfate (6 M) and hydroquinone (10 mM) in a 60 °C incubator for 30 min following by the automated process. We used 60 µL of ccfDNA for bisulfite conversion, and the bisulfite-converted ccfDNA was eluted in 100 µL of molecular biology-grade water. The automated sample process was performed using a 24 deep-well plate (ThermoFisher Scientific, Vantaa, Finland, 95040470). The eluted bisulfite-converted ccfDNA was immediately used for methylation-specific real-time PCR.

### Automatic Circulating Cell-Free DNA Extraction Using LabTurbo 24C

The automated ccfDNA extraction process was performed using the LabTurbo 24 Compact System (Taigen Bioscience Co., Taipei, Taiwan) according to the manufacturer's instructions. The workflow followed the instruction manual supplied with the Labturbo Circulating DNA mini kit (Cat No. AIOLCD1600, Taigen Bioscience Co., Taipei, Taiwan), with full automation of vacuum-based DNA extraction of up to 24 samples simultaneously. The ccfDNA was extracted from 1.6 mL of plasma and eluted in 60 µL of molecular biology-grade water (46-000-CM, Corning, NY, USA).

### MethylationEPIC BeadChip Array for Genome-Wide Methylation Analysis

The MethylationEPIC BeadChip (EPIC) array covers 850,000 CpG sites, including >90% of the CpGs and 99% Refseq genes from HM450 and an additional 413,743 CpGs. The EPIC array has been validated in comparison to the 450K platform for blood samples. The genome-wide methylation analysis was performed using the Infinium^{®} MethylationEPIC BeadChip array (Illumina, San Diego, CA, USA). Bisulfite conversion was performed for 500 ng of DNA using the EpiTect Fast DNA Bisulfite Kit (QIAGEN, Bonn, Germany, Cat. No. 59826) according to the manufacturer's instructions. Methylation scores for each CpG site are represented as "beta" values ranging from 0 (unmethylated) to 1 (fully methylated) by determining the ratios of the methylated signal intensities to the sums of the methylated and unmethylated signal outputs. Infinium MethylationEPIC BeadChip data were analyzed using GenomeStudio Methylation Module version 2011.1. The Infinium MethylationEPIC BeadChip employs both Infinium I and Infinium II assays. The Infinium I assay design employs 2 bead types per CpG locus, 1 each for the methylated and unmethylated states. The Infinium II design uses 1 bead type, with the methylated state determined at the single base extension step after hybridization (right panel). A differentially methylated CpG heatmap of the target genes was visualized by a heatmap using heatmapper software. A gradient-scale heatmap was used to visualize the DNA methylation level from low to high.

### Probe-Based Quantitative Methylation-Specific PCR (qMSP)

After bisulfite conversion of DNA, which was done according to the manufacturer's recommended protocol, the DNA methylation levels of *TMEM240, MROH6, BEND5,* and *SMAD3* were measured using TaqMan quantitative methylation-specific PCR (qMSP) with a LightCycler 96 (Roche Applied Science, Penzberg, Germany). qMSP was performed using the SensiFAST^{™} Probe No-ROX Kit (Bioline, London, UK, Cat. No. BIO-86020) with specific primers and methyl-TaqMan probes of candidate genes. Normalized DNA methylation values, which were calibrated to the control group, were obtained using LightCycler Relative Quantification software (Version 1.5, Roche Applied Science). The beta-actin (ACTB) gene was used as methylation-independent DNA control. The primers/probes for the ACTB gene were designed without the CpG site (as a control for input DNA). The primers/probes for candidate genes were designed on their methylated promotor regions, especially on the identified differential regions between normal and tumor tissues. According to the sequencing results, only when all CpG sites are methylated can a successful PCR reaction occur. The target genes were considered hypermethylated when the methylation level relative to that of the ACTB gene was at least 2-fold higher in the colorectal tumor compared with the paired normal colorectal tissue sample. The specificity of the candidate gene methylation end products was confirmed by bisulfite sequencing. The primers and probes used for qMSP are listed in Table 1.

### Statistical Analysis

The Pearson's chi-squared test, Mann-Whitney U test, Wilcoxon test and Spearman's rank correlation analyses were performed using SPSS (IBM, Armonk, NY, USA). The Pearson's chi-squared test was used to compare colorectal cancer patients in terms of candidate gene methylation, RNA expression and other clinical data. The paired-sample Wilcoxon test and t-test was used to compare differences in DNA methylation between tumors and matched adjacent normal tissues, different cancer types, as well as in candidate ccfDNA methylation between surgery treatment in colorectal cancer patients. The Spearman's rank correlation was adopted to analyze the methylation levels of the tumor and plasma samples.

To assess multiple biomarkers, Kang's nonparametric stepwise classification method was employed to evaluate the accuracy in identifying colorectal cancer patients when the proposed gene biomarkers were used. In addition to the accuracy, other commonly used measures for evaluating the classification, such as the area under the receiver operating characteristic curve (AUC), sensitivity, specificity, false-positive rate and false-negative rate, were also reported.

### Example 1 Methylation Status of Target DNA Sequence in Colorectal Cancer Tissue

The β value for Illumina Methylation 450K array-based data was generated from The Cancer Genome Atlas (TCGA) Research Network. The target nucleic acid and genes were selected when β value from normal tissues is less than 0.15; Δβ value (the value of Tumor subtracts that of normal tissues) is higher than 0.5 or lower than 0.25. The methylation statuses Δβ value (T)of the target DNA sequences are shown in Table 2.

**Table 2**

| | MROH6 | TMEM240 | BEND5 | SMAD3 |
|---|---|---|---|---|
| Colorectal cancer | 0.50 | 0.73 | 0.53 | 0.09 |

β value (T), methylation status of tumor tissues; β value(T) ≥ 0.5 will be calculated as hypermethylation biomarkers and β value(T) ≤ 0.25 will be calculated as hypomethylation biomarker.

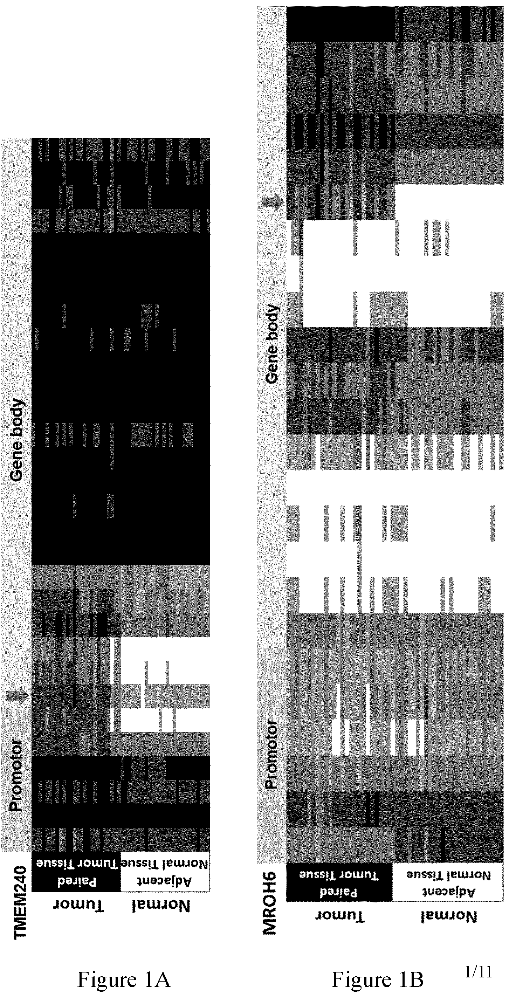

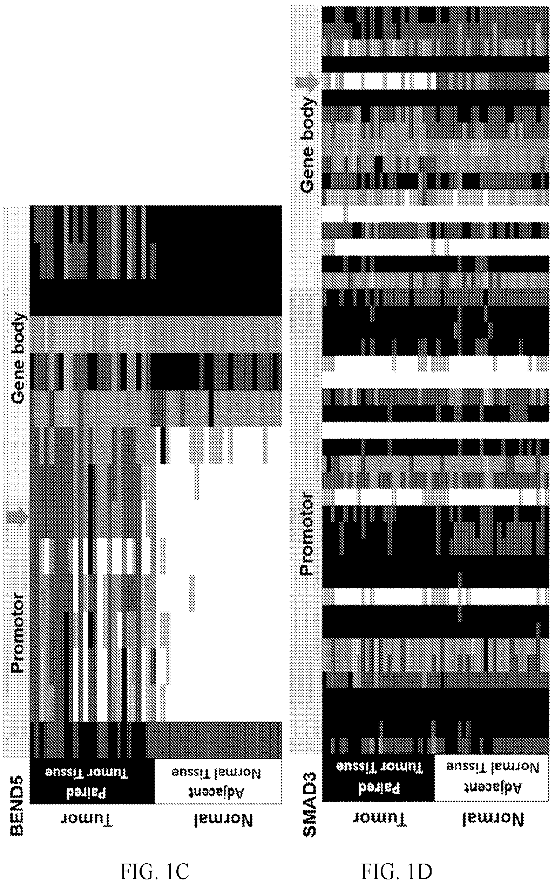

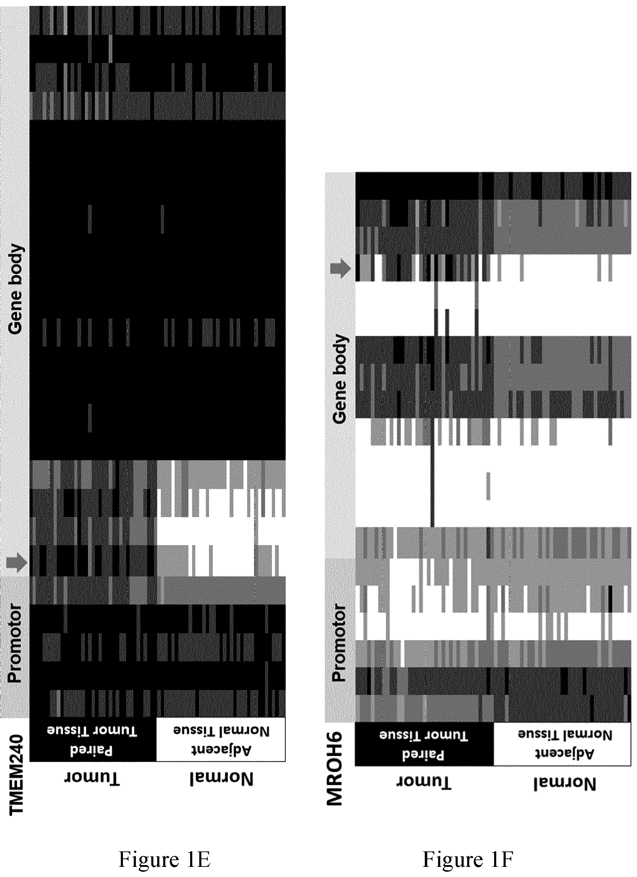

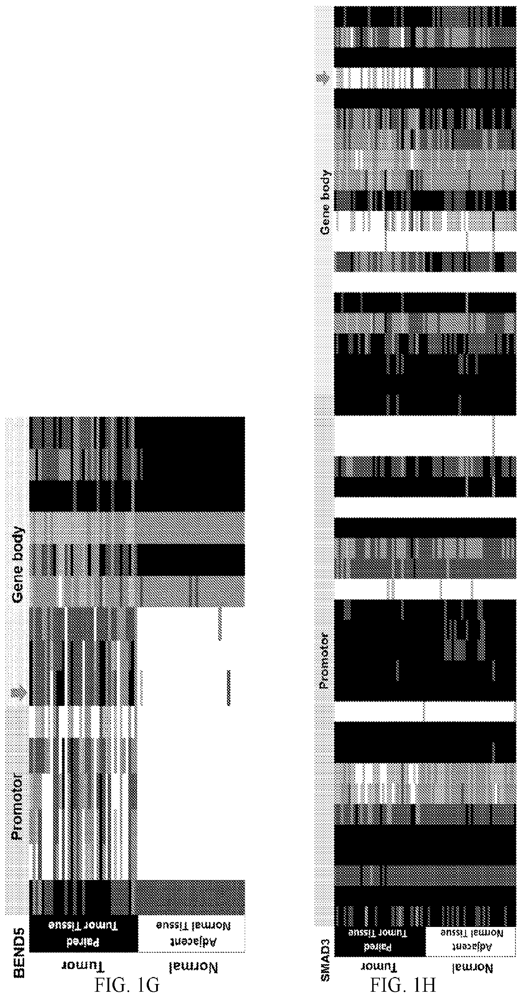

Figure 1 shows the difference of methylation status (β value) of target nucleic acid and genes between tumor tissues and adjacent normal tissues (n=97). Darker color indicates tissues with higher methylation status according to Illumina Methylation 450K array-based data.

### Example 2 Early Detection of Methylation Status of Epigenetic Biomarkers of Target Genes in Plasma Samples of Healthy Subjects and Colorectal Cancer Patients

The circulating cell-free DNA was extracted from plasma. Briefly, 3.5 mL of plasma was isolated immediately from 10 mL of peripheral blood. After circulating cell-free DNA (cfDNA) was extracted from plasma that obtained from colorectal cancer patients and healthy subjects, cfDNA was performed by bisulfite conversion. Probe-based methylation specific real-time PCR (qMSP) was used for cfDNA methylation analyses.

Data obtained from the qMSP assays was processed according to the following criteria. If the Cₜ value of qMSP was less than 45 cycles for circulating methylated *TMEM240* gene; less than 40 cycles for circulating methylated *MROH6* gene; less than 45 cycles for circulating methylated *BEND5*; higher than 45 cycles for circulating methylated *SMAD3,* a score was defined as 1, respectively. Otherwise, a score was defined as 0.

The average of the total scores of qMSP in *TMEM240, MROH6,* and *BEND5* was higher than 0.2, the human subject was defined as CRC patient.

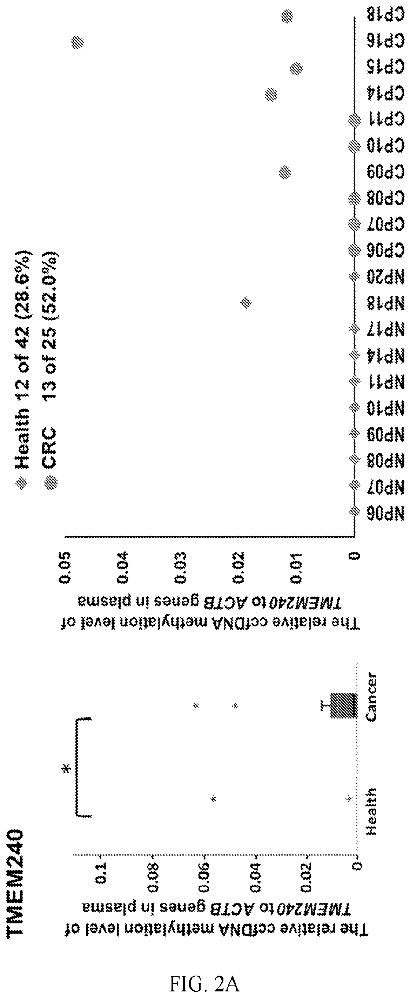

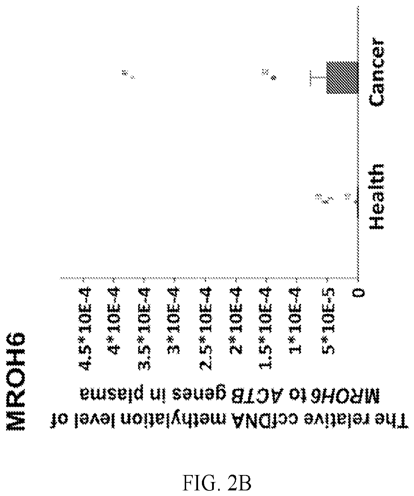

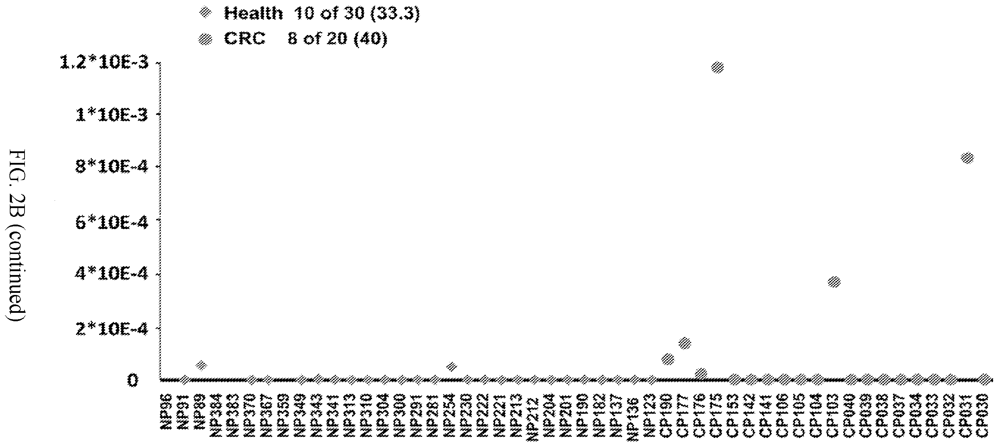

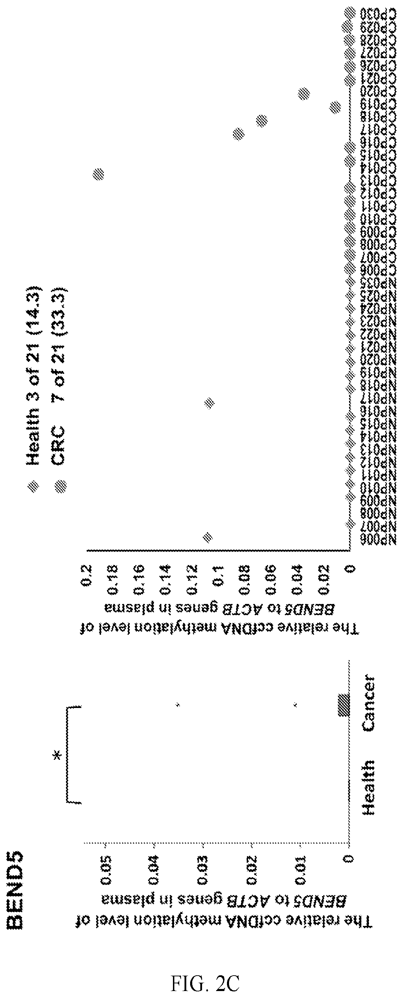

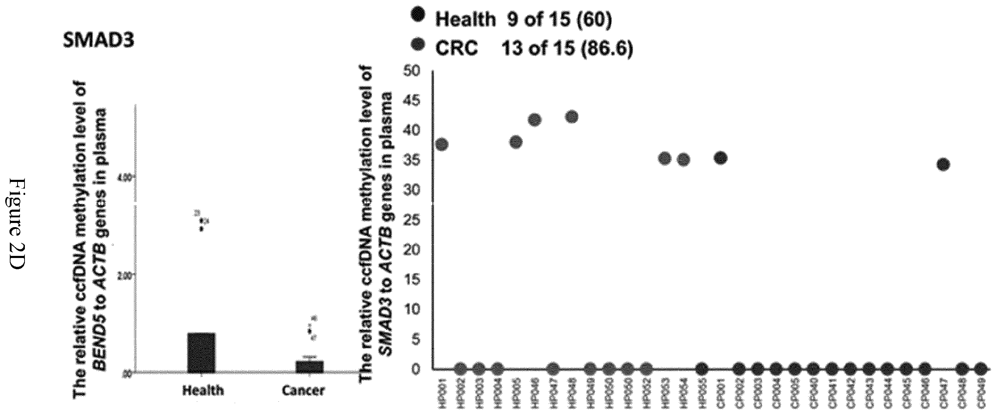

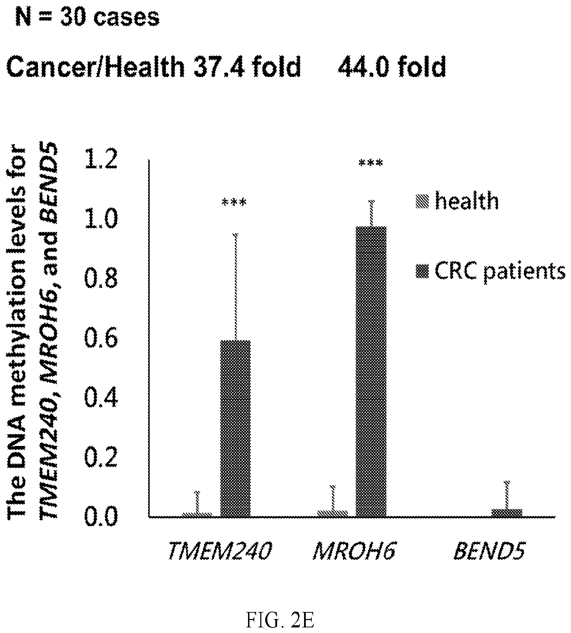

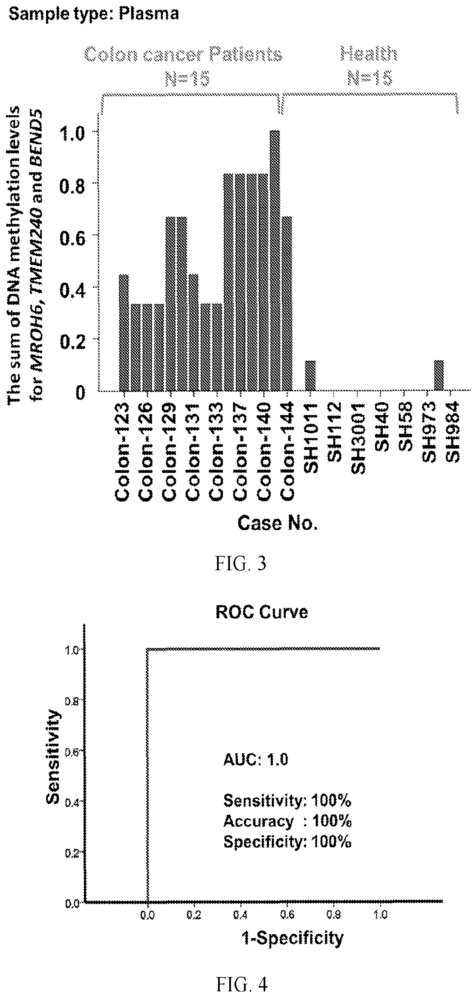

Figure 2 shows the difference in early detection of the methylation status of epigenetic biomarkers of target genes in plasma samples of healthy subjects and colorectal cancer patients. Figure 3 shows the sum of DNA methylation levels (scores) of epigenetic biomarkers of target genes in plasma samples of healthy subjects and colorectal cancer patients. In addition, Receiver Operating Characteristic (ROC) Curve Analysis as shown in Figure 4 indicates the early detection of the methylation status of epigenetic biomarkers of target genes in colorectal cancer patients and healthy subjects.

## Claims

1. A method for early diagnosing colorectal cancer in a subject, comprising (a) providing a biological sample from the subject, and (b) determining the methylation status of a target DNA sequence comprising *MROH6* in the biological sample, wherein the presence of hypermethylation in *MROH6* of the subject is indicative of colorectal cancer.

2. The method of claim 1, wherein the target DNA sequence further comprises one or more DNA sequences selected from the group consisting of *TMEM240, BEND5* and *SMAD3,* or any of combinations thereof; wherein the presence of hypermethylation in *TMEM240,* hypermethylation in *BEND5,* or hypomethylation in *SMAD3,* of the subject is indicative of colorectal cancer.

3. The method of claim 1 or 2, wherein the biological sample is a tissue, cell, blood, urine, serum, plasma, stool, ascites, sputum, saliva, gastric juice, bile, or oral mucosa.

4. The method of claim 2, wherein the method further comprises a step of defining a score as 1 if the presence of hypermethylation or hypomethylation of each gene and, defining a score as 0 if the absence of hypermethylation or hypomethylation of each gene, and summing the score.

5. The method of any of the preceding claims, wherein the methylation status is determined by a polymerase chain reaction; a target DNA sequence methylation specific primer for *MROH6* is used in the methylation determination; and the target DNA sequence methylation specific primer for *MROH6* has an identity of about 85% to a sequence selected from the group consisting of SEQ ID NOs: 5, and 6.

6. The method of any of the preceding claims, wherein a target DNA sequence methylation specific probe for *MROH6* is used in the methylation determination, and the target DNA sequence methylation specific probe for *MROH6* has an identity of about 85% to a sequence of SEQ ID NO: 7.

7. The method of claim 2, wherein the target DNA sequence comprises any of the following combinations of the DNA sequences: *TMEM240* and *MROH6; TMEM240, MROH6* and *BEND5, TMEM240, MROH6, BEND5* and *SMAD3; TMEM240, BEND5* and *SMAD3; TMEM240, MROH6,* and *SMAD3; MROH6* and *BEND5;* and *MROH6, BEND5* and *SMAD3.*

8. The method of claim 2, wherein a target DNA sequence methylation specific primer for *TMEM240, BEND5,* or *SMAD3* or a combination thereof is used in the methylation determination, and wherein the target DNA sequence methylation specific primer for *TMEM240* has an identity of about 85% to a sequence selected from the group consisting of SEQ ID NOs: 1, 2, and 3; the target DNA sequence methylation specific primer for *BEND5* has an identity of about 85% to a sequence selected from the group consisting of SEQ ID NOs: 8 to 11; and the target DNA sequence methylation specific primer for *SMAD3* has an identity of about 85% to a sequence selected from the group consisting of SEQ ID NOs: 14 to 15.

9. The method of claim 2, wherein a target DNA sequence methylation specific probe for *TMEM240, BEND5,* or *SMAD3* or a combination thereof is used in the methylation determination, and wherein the target DNA sequence methylation specific probe for *TMEM240* has an identity of about 85% to a sequence of SEQ ID NO: 4; the target DNA sequence methylation specific probe for *BEND5* has an identity of about 85% to a sequence selected from the group consisting of SEQ ID NOs: 12 to 13; and the target DNA sequence methylation specific probe for *SMAD3* has an identity of about 85% to a sequence of SEQ ID NO: 16.

10. An isolated nucleic acid molecule having a sequence selected from the group consisting of: SEQ ID Nos: 5 to 7

11. A kit for early diagnosing colorectal cancer in a subject, which comprises a target DNA sequence methylation specific primer pair for detection of a methylation status of a target DNA sequence comprising *MROH6,* wherein the target DNA sequence methylation specific primer pair for *MROH6* comprises SEQ ID NOs: 5 and 6.

12. The kit of claim 11, further comprising a target DNA sequence methylation specific probe for detection of a methylation status of a target DNA sequence comprising *MROH6,* wherein the target DNA sequence methylation specific probe for *MROH6* comprises SEQ ID NO: 7

13. The kit of claim 11 or 12, wherein the target DNA sequence further comprises *TMEM240, BEND5,* or *SMAD3,* and the kit further comprises one or more target DNA sequence methylation specific primer pairs for detection of a methylation status of *TMEM240, BEND5,* or *SMAD3,* or any combination thereof; and wherein the target DNA sequence methylation specific primer pair for *TMEM240* comprises SEQ ID NOs: 1 and 2 or 1 and 3; the target DNA sequence methylation specific primer pair for *BEND5* comprises SEQ ID Nos. 8 and 9 or 10 and 11; and the target DNA sequence methylation specific primer pair for *SMAD3* comprises SEQ ID Nos. 14 and 15.

14. The kit of any of claims 11 to 13, wherein the kit further comprises one or more target DNA sequence methylation specific probe for detection of a methylation status of *TMEM240, BEND5,* or *SMAD3,* or any combination thereof, and wherein the target DNA sequence methylation specific probe for *TMEM240* comprises SEQ ID NO: 4; the target DNA sequence methylation specific probe for *BEND5* comprises SEQ ID NO: 12 or 13; and the target DNA sequence methylation specific probe for *SMAD3* comprises SEQ ID NO: 16.

## Patentansprüche

1. Verfahren zur Frühdiagnose von kolorektalem Krebs bei einem Subjekt, umfassend (a) Bereitstellen einer biologischen Probe von dem Subjekt und (b) Bestimmen des Methylierungsstatus einer Ziel-DNA-Sequenz umfassend *MROH6* in der biologischen Probe, wobei das Vorhandensein von Hypermethylierung in *MROH6* des Subjekts indikativ für kolorektalen Krebs ist.

2. Verfahren nach Anspruch 1, wobei die Ziel-DNA-Sequenz ferner eine oder mehrere DNA-Sequenzen umfasst, die ausgewählt sind aus der Gruppe, bestehend aus *TMEM240, BEND5* und *SMAD3* oder beliebigen Kombinationen davon; wobei das Vorhandensein einer Hypermethylierung in *TMEM240,* einer Hypermethylierung in *BEND5* oder einer Hypomethylierung in *SMAD3* bei dem Patienten indikativ für kolorektalen Krebs ist.

3. Verfahren nach Anspruch 1 oder 2, wobei die biologische Probe ein Gewebe, eine Zelle, Blut, Urin, Serum, Plasma, Stuhl, Aszites, Sputum, Speichel, Magensaft, Galle oder Mundschleimhaut ist.

4. Verfahren nach Anspruch 2, wobei das Verfahren ferner einen Schritt eines Definierens eines Punktwerts als 1 in Anwesenheit von Hypermethylierung oder Hypomethylierung jedes Gens, und eines Definierens eines Punktwerts als 0 in Abwesenheit von Hypermethylierung oder Hypomethylierung jedes Gens, und eines Summierens des Punktwerts umfasst.

5. Verfahren nach einem der vorherigen Ansprüche, wobei der Methylierungsstatus durch eine Polymerase-Kettenreaktion bestimmt wird; bei der Methylierungsbestimmung ein methylierungsspezifischer Ziel-DNA-Sequenz-Primer für *MROH6* verwendet wird; und der methylierungsspezifische Ziel-DNA-Sequenz-Primer für *MROH6* eine Identität von etwa 85 % mit einer Sequenz aufweist, die ausgewählt ist aus der Gruppe, bestehend aus SEQ ID NO: 5 und 6.

6. Verfahren nach einem der vorherigen Ansprüche, wobei bei der Methylierungsbestimmung eine methylierungsspezifische Ziel-DNA-Sequenzsonde für *MROH6* verwendet wird und die methylierungsspezifische Ziel-DNA-Sequenzsonde für *MROH6* eine Identität von etwa 85 % mit einer Sequenz von SEQ ID NO: 7 aufweist.

7. Verfahren nach Anspruch 2, wobei die Ziel-DNA-Sequenz eine beliebige der folgenden Kombinationen von DNA-Sequenzen umfasst: *TMEM240* und *MROH6; TMEM240, MROH6* und *BEND5; TMEM240, MROH6, BEND5* und *SMAD3; IMEM240, BEND5* und *SMAD3; TMEM240, MROH6* und *SMAD3; MROH6* und *BEND5;* und *MROH6, BEND5* und *SMAD3.*

8. Verfahren nach Anspruch 2, wobei bei der Methylierungsbestimmung ein methylierungsspezifischer Ziel-DNA-Sequenz-Primer für *TMEM240, BEND5* oder *SMAD3* oder eine Kombination davon verwendet wird, und wobei der methylierungsspezifische Ziel-DNA-Sequenz-Primer für *TMEM240* eine Identität von etwa 85 % mit einer Sequenz aufweist, die ausgewählt ist aus der Gruppe, bestehend aus SEQ ID NO: 1, 2 und 3; der methylierungsspezifische Ziel-DNA-Sequenz-Primer für *BEND5* eine Identität von etwa 85 % mit einer Sequenz aufweist, die ausgewählt ist aus der Gruppe, bestehend aus SEQ ID NO: 8 bis 11; und der methylierungsspezifische Ziel-DNA-Sequenz-Primer für *SMAD3* eine Identität von etwa 85 % mit einer Sequenz aufweist, die ausgewählt ist aus der Gruppe, bestehend aus SEQ ID NO: 14 bis 15.

9. Verfahren nach Anspruch 2, wobei bei der Methylierungsbestimmung eine ziel-DNA-Sequenz-methylierungsspezifische Sonde für *TMEM240, BEND5* oder *SMAD3* oder eine Kombination davon verwendet wird, und wobei die Ziel-DNA-Sequenz-methylierungsspezifische Sonde für *TMEM240* eine Identität von etwa 85 % mit einer Sequenz von SEQ ID NO: 4 aufweist; die Ziel-DNA-Sequenz-methylierungsspezifische Sonde für *BEND5* eine Identität von etwa 85 % mit einer Sequenz aufweist, die ausgewählt ist aus der Gruppe, bestehend aus SEQ ID NO: 12 bis 13; und die Ziel-DNA-Sequenz-methylierungsspezifische Sonde für *SMAD3* eine Identität von etwa 85 % mit einer Sequenz von SEQ ID NO: 16 aufweist.

10. Isoliertes Nukleinsäuremolekül, das eine Sequenz aufweist, die ausgewählt ist aus der Gruppe, bestehend aus: SEQ ID NO: 5 bis 7.

11. Kit zur Frühdiagnose von kolorektalem Krebs bei einem Subjekt, das ein für eine Ziel-DNA-Sequenz methylierungsspezifisches Primerpaar zum Nachweis eines Methylierungsstatus einer *MROH6* umfassenden Ziel-DNA-Sequenz umfasst, wobei das für eine Ziel-DNA-Sequenz methylierungsspezifische Primerpaar für *MROH6* die SEQ ID NO: 5 und 6 umfasst.

12. Kit nach Anspruch 11, ferner umfassend eine methylierungsspezifische Ziel-DNA-Sequenz-Sonde zum Nachweis eines Methylierungsstatus einer *MROH6* umfassenden Ziel-DNA-Sequenz, wobei die methylierungsspezifische Ziel-DNA-Sequenz-Sonde für *MROH6* SEQ ID NO: 7 umfasst.

13. Kit nach Anspruch 11 oder 12, wobei die Ziel-DNA-Sequenz ferner *TMEM240, BEND5* oder *SMAD3* umfasst , und das Kit ferner ein oder mehrere Ziel-DNA-Sequenz-methylierungsspezifische Primerpaare zum Nachweis eines Methylierungsstatus von *TMEM240, BEND5* oder *SMAD3* oder einer beliebigen Kombination davon umfasst; und wobei das Ziel-DNA-Sequenz-methylierungsspezifische Primerpaar für *TMEM240* die SEQ ID NO: 1 und 2 oder 1 und 3 umfasst; das für die DNA-Zielsequenz-Methylierung spezifische Primerpaar für *BEND5* die SEQ ID NO. 8 und 9 oder 10 und 11 umfasst; und das für die Ziel-DNA-Sequenz-methylierungsspezifische Primerpaar für *SMAD3* SEQ ID Nr. 14 und 15 umfasst.

14. Kit nach einem der Ansprüche 11 bis 13, wobei der Kit ferner eine oder mehrere ziel-DNA-Sequenz-methylierungsspezifische Sonde(n) zum Nachweis eines Methylierungsstatus von *TMEM240, BEND5* oder *SMAD3* oder einer beliebigen Kombination davon umfasst, und wobei die ziel-DNA-Sequenz-methylierungsmspezifische Sonde für *TMEM240* SEQ ID NO: 4 umfasst; die Ziel-DNA-Sequenz-methylierungsspezifische Sonde für *BEND5* SEQ ID NO: 12 oder 13 umfasst; und die Ziel-DNA-Sequenz-methylierungsspezifische Sonde für *SMAD3* SEQ ID NO: 16 umfasst.

## Revendications

1. Procédé de diagnostic précoce du cancer colorectal chez un sujet, comprenant (a) la fourniture d'un échantillon biologique provenant du sujet, et (b) la détermination de l'état de méthylation d'une séquence d'ADN cible comprenant *MROH6* dans l'échantillon biologique, la présence d'une hyperméthylation dans *MROH6* du sujet étant indicative d'un cancer colorectal.

2. Procédé selon la revendication 1, dans lequel la séquence d'ADN cible comprend en outre une ou plusieurs séquences d'ADN choisies dans le groupe constitué par *TMEM240, BEND5* et *SMAD3,* ou toute combinaison de ces séquences ; dans lequel la présence d'une hyperméthylation dans *TMEM240,* d'une hyperméthylation dans *BEND5* ou d'une hypométhylation dans *SMAD3,* chez le sujet, est indicative d'un cancer colorectal.

3. Procédé selon la revendication 1 ou 2, dans lequel l'échantillon biologique est un tissu, une cellule, du sang, de l'urine, du sérum, du plasma, des selles, des ascites, des expectorations, de la salive, du suc gastrique, de la bile ou de la muqueuse buccale.

4. Procédé selon la revendication 2, dans lequel le procédé comprend en outre une étape consistant à définir un score de 1 en cas de présence d'hyperméthylation ou d'hypométhylation de chaque gène et à définir un score de 0 en cas d'absence d'hyperméthylation ou d'hypométhylation de chaque gène, et à additionner les scores.

5. Procédé selon l'une quelconque des revendications précédentes, dans lequel l'état de méthylation est déterminé par une réaction en chaîne de la polymérase ; une amorce spécifique de la méthylation de la séquence d'ADN cible pour *MROH6* est utilisée dans la détermination de la méthylation ; et l'amorce spécifique de la méthylation de la séquence d'ADN cible *pour MROH6* présente une identité d'environ 85 % avec une séquence choisie dans le groupe constitué de SEQ ID N° : 5 et 6.

6. Procédé selon l'une des revendications précédentes, dans lequel une sonde spécifique de méthylation de la séquence d'ADN cible pour *MROH6* est utilisée dans la détermination de la méthylation, et la sonde spécifique de méthylation de la séquence d'ADN cible pour *MROH6* présente une identité d'environ 85 % avec une séquence de SEQ ID N° : 7.

7. Procédé selon la revendication 2, dans lequel la séquence d'ADN cible comprend l'une des combinaisons suivantes de séquences d'ADN : *TMEM240* et *MROH6; TMEM240, MROH6* et *BEND5 ; TMEM240, MROH6, BEND 5 et SMAD3 ; IMEM240, BEND5* et *SMAD3 ; TMEM240, MROH6* et *SMAD3 ; MROH6* et *BEND5 ; et MROH6, BEND5* et *SMAD3.*

8. Procédé selon la revendication 2, dans lequel une amorce spécifique de méthylation de la séquence d'ADN cible pour *TMEM240, BEND5,* ou *SMAD3* ou une combinaison de ceux-ci est utilisée dans la détermination de la méthylation, et dans lequel l'amorce spécifique de méthylation de la séquence d'ADN cible pour *TMEM240* présente une identité d'environ 85 % avec une séquence choisie dans le groupe constitué de SEQ ID N° : 1, 2 et 3 ; l'amorce spécifique de méthylation de la séquence d'ADN cible pour *BEND5* présente une identité d'environ 85 % avec une séquence choisie dans le groupe constitué des SEQ ID N° : 8 à 11 ; et l'amorce spécifique de méthylation de la séquence d'ADN cible pour *SMAD3* présente une identité d'environ 85 % par rapport à une séquence choisie dans le groupe constitué de SEQ ID N° : 14 à 15.

9. Procédé selon la revendication 2, dans lequel une sonde spécifique de méthylation de la séquence d'ADN cible pour *TMEM240, BEND5,* ou *SMAD3* ou une combinaison de ceux-ci est utilisée dans la détermination de la méthylation, et dans lequel la sonde spécifique de méthylation de la séquence d'ADN cible pour *TMEM240* présente une identité d'environ 85 % avec une séquence de SEQ ID NO : 4 ; la sonde spécifique de méthylation de la séquence d'ADN cible pour *BEND5* présente une identité d'environ 85 % avec une séquence choisie dans le groupe constitué des SEQ ID N° : 12 à 13 ; et la sonde spécifique de méthylation de la séquence d'ADN cible pour *SMAD3* présente une identité d'environ 85 % avec une séquence de SEQ ID N° : 16.

10. Molécule d'acide nucléique isolée présentant une séquence choisie dans le groupe constitué par : SEQ ID N° : 5 à 7.

11. Kit pour le diagnostic précoce du cancer colorectal chez un sujet, qui comprend une paire d'amorces spécifiques de méthylation de la séquence d'ADN cible pour la détection d'un état de méthylation d'une séquence d'ADN cible comprenant *MROH6*, dans lequel la paire d'amorces spécifiques de méthylation de la séquence d'ADN cible *pour MROH6* comprend les SEQ ID N° : 5 et 6.

12. Kit selon la revendication 11, comprenant en outre une sonde spécifique de méthylation de la séquence d'ADN cible pour la détection d'un état de méthylation d'une séquence d'ADN cible comprenant *MROH6*, dans lequel la sonde spécifique de méthylation de la séquence d'ADN cible pour *MROH6* comprend la SEQ ID N° : 7.

13. Kit selon la revendication 11 ou 12, dans lequel la séquence d'ADN cible comprend en outre *TMEM240, BEND5* ou *SMAD3,* et le kit comprend en outre une ou plusieurs paires d'amorces spécifiques de la méthylation de la séquence d'ADN cible pour la détection d'un état de méthylation de *TMEM240, BEND5* ou *SMAD3,* ou toute combinaison de ceux-ci ; et dans lequel la paire d'amorces spécifiques de la méthylation de la séquence d'ADN cible pour *TMEM240* comprend les SEQ ID N° : 1 et 2 ou 1 et 3 ; la paire d'amorces spécifique de la méthylation de la séquence d'ADN cible pour *BEND5* comprend les SEQ ID N° 8 et 9 ou 10 et 11 ; et la paire d'amorces spécifique de la méthylation de la séquence d'ADN cible pour *SMAD3* comprend les SEQ ID N° 14 et 15.

14. Kit selon l'une des revendications 11 à 13, dans lequel le kit comprend en outre une ou plusieurs sondes spécifiques de méthylation de la séquence d'ADN cible pour la détection d'un état de méthylation de *TMEM240, BEND5,* ou *SMAD3,* ou toute combinaison de ceux-ci, et dans lequel la sonde spécifique de méthylation de la séquence d'ADN cible pour *TMEM240* comprend la SEQ ID N° : 4 ; la sonde spécifique de méthylation de la séquence d'ADN cible pour *BEND5* comprend la SEQ ID N° : 12 ou 13 ; et la sonde spécifique de méthylation de la séquence d'ADN cible pour SMAD3 comprend la SEQ ID N° : 16.
